# EUROPEAN PATENT SPECIFICATION

(11) **EP 1 381 358 B1**
(45) Date of publication and mention of the grant of the patent: **03.05.2006**
(21) Application number: 02718317.7
(22) Date of filing: 27.03.2002
(51) Int. Cl.: A61K 31/277, A61K 9/14, A61P 35/00

(54) **SOLID PHARMACEUTICAL COMPOSITION COMPRISING 4'-CYANO-TRIFLUORO-3-(4-FLUOROPHENYLSULPHONYL) -2-HYDROXY-2-METHYLPROPIONO- M TOLUIDIDE AND PVP**
FESTE ARZNEIZUSAMMENSETZUNG ENTHALTEND 4'-CYANO-TRIFLUORO-3-(4-FLUOROPHENYLSULPHONYL) -2-HYDROXY-2-METHYLPROPIONO- M TOLUIDIDE UND PVP
COMPOSITION PHARMACEUTIQUE SOLIDE CONTENANT 4-CYANO-TRIFLUORO-3-(4-FLUOROPHENYLSULFONYL)-2-HYDROXY-2-METHYLPROPIONO-M-TOLUIDIURE ET PVP

(30) Priority: 02.04.2001 SE 0101171; 04.09.2001 SE 0102957; 25.10.2001 SE 0103565
(43) Date of publication of application: 21.01.2004
(73) Proprietor: AstraZeneca AB, 151 85 (SE)
(72) Inventor: CAHILL, Julie, Kay, Macclesfield, Cheshire SK10 4TG (GB); BATEMAN, Nicola, Frances, Macclesfield, Cheshire SK10 4TG (GB)
(74) Representative: Gainey, Laurence David Scott
(86) International application number: PCT/GB2002/001439
(87) International publication number: WO 2002/080902

(56) References cited:
- EP-A- 1 027 886
- WO-A-01/49294
- WO-A-95/19770
- DENIS L ET AL: "PHARMACODYNAMICS AND PHARMACOKINETICS OF BICALUTAMIDE: DEFINING AN ACTIVE DOSING REGIMEN" UROLOGY, BELLE MEAD, NJ, US, vol. 47, no. SUPPL 1A, 1996, pages 26-28, XP000605159 ISSN: 0090-4295

## Description

The present invention relates to a pharmaceutical formulation comprising bicalutamide (with the chemical name: 4'-cyano-α',α',α'-trifluoro-3-(4-fluorophenylsulphonyl)-2-hydroxy-2-methylpropiono-*m*-toluidide) in a solid dispersion with PVP. In one embodiment, >50% of the 4'-cyano-α',α',α'-trifluoro-3-(4-fluorophenylsulphonyl)-2-hydroxy-2-methylpropiono-*m*-toluidide is provided in the form of the R-enantiomer. The invention also relates to a daily pharmaceutical dose of 4'-cyano-α',α',α'-trifluoro-3-(4-fluorophenylsulphonyl)-2-hydroxy-2-methylpropiono-*m*-toluidide provided by such a formulation. In addition, the invention relates to the use of PVP in solid dispersion with 4'-cyano-α',α',α'-trifluoro-3-(4-fluorophenylsulphonyl)-2-hydroxy-2-methylpropiono-*m-*toluidide (in one embodiment wherein >50% of the 4'-cyano-α',α',α'-trifluoro-3-(4-fluorophenylsulphonyl)-2-hydroxy-2-methylpropiono-*m*-toluidide is provided in the form of the R-enantiomer) for increasing the bioavailability of the 4'-cyano-α',α',α'-trifluoro-3-(4-fluorophenylsulphonyl)-2-hydroxy-2-methylpropiono-*m*-toluidide; for reducing inter-patient variability in plasma concentrations of 4'-cyano-α',α',α'-trifluoro-3-(4-fluorophenylsulphonyl)-2-hydroxy-2-methylpropiono-*m*-toluidide; for enhancing the storage stability of the 4'-cyano-α',α',α'-trifluoro-3-(4-fluorophenylsulphonyl)-2-hydroxy-2-methylpropiono-*m*-toluidide; or for treating and/or reducing the risk of prostate cancer in a patient.

### BACKGROUND TO THE INVENTION

Bicalutamide, a non-steroidal anti-androgen, is the racemate of 4'-cyano-α',α',α'-trifluoro-3-(4-fluorophenylsulphonyl)-2-hydroxy-2-methylpropiono-*m*-toluidide. Bicalutamide is known by the AstraZeneca trade name CASODEX™. EP-100172 discloses 4'-cyano-α',α',α'-trifluoro-3-(4-fluorophenylsulphonyl)-2-hydroxy-2-methylpropiono-*m-*toluidide (named in EP-100172 as 4-cyano-3-trifluoromethyl-*N*-(3-*p*-fluorophenylsulphonyl-2-hydroxy-2-methylpropionyl)aniline) as the 8^{th} compound listed in the table in Example 6. The corresponding structure is shown in formula I:-

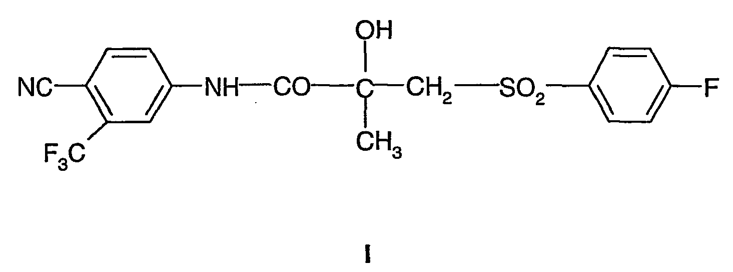

Bicalutamide can be used to combat prostate cancer. The properties and usefulness of bicalutamide as an anti-androgen have been reviewed in B J A Furr *et al.,* Urology, 1996, 47 (Suppl. 1A), 13-25, and G J C Kolvenbag *et al.,* Urology, 1996, 47 (Suppl. 1A) 70-79. 4'-cyano-α',α',α'-trifluoro-3-(4-fluorophenylsulphonyl)-2-hydroxy-2-methylpropiono-*m-*toluidide can exist in distinct R- and S- enantiomeric forms. The R-enantiomer is the (-) isomer and is the pharmacologically active compound *in vivo.* For further details of the enantiomers, reference is made to Tucker and Chesterton, *J. Med. Chem.* 31, pp 885-887 (1988).

The chemical synthesis of racemic 4'-cyano-α',α',α'-trifluoro-3-(4-fluorophenylsulphonyl)-2-hydroxy-2-methylpropiono-*m*-toluidide is described in US4636505, and this disclosure is incorporated herein by reference. The R-enantiomer may be obtained by the resolution of enantiomers from the racemate or resolution of precursors of the enantiomers using fractional crystallisation or chromatographic separation of diastereomeric esters of chiral acids. Other methods will, however, be evident to the skilled addressee using routine techniques for the preparation of enantiomers. For example, the R-enantiomer may be prepared by simple crystallisation and chromatographic resolution (see, for example, Wilen and Lochmuller, "Tables of Resolving Agents", *J. Chromatography,* 113, 283-302 (1975) and E L Eliel, *Stereochemistry of Carbon Compounds,* McGraw Hill (1962)). Another method involves resolution of the carboxylic acid precursor, 3-(4-fluorophenyl)-2-hydroxy-2-methylpropanoic acid, by fractional crystallisation of diastereomeric salts with chiral amines. The Tucker and Chesterton reference cited above discloses the chromatographic separation of the R-and S- enantiomers from racemic 4'-cyano-α',α',α'-trifluoro-3-(4-fluorophenylsulphonyl)-2-hydroxy-2-methylpropiono-*m*-toluidide. The method involves the chromatographic separation of R-camphanoyl esters of the racemate and their hydrolysis and oxidation to the R- and S-enantiomers. This disclosure provide an illustration of a method of obtaining the enantiomers for use in the present invention.

Bicalutamide (4'-cyano-α',α',α'-trifluoro-3-(4-fluorophenylsulphonyl)-2-hydroxy-2-methylpropiono-*m*-toluidide racemate) is used in conventional oral tablet form (eg, at a daily monotherapy dose of 150mg) to combat prostate cancer in men. The bioavailability of the bicalutamide to the patient is determined to a certain extent by the dissolution rate and solubility of the drug in the GI tract, which affects absorption across mucosal membranes in the GI tract. The relative bioavailability of bicalutamide for a series of formulations can be assessed by determining the area under the curve (AUC) of a graph of plasma bicalutamide concentration v. time elapsed since administration of the bicalutamide. As a consequence of sub-optimal rates of dissolution and degree of solubility of the drug, there is observed a high degree of inter-patient variability in the bioavailability of bicalutamide administered in conventional tablet form. This may result in sub-optimal treatment efficacy in a proportion of patients. In addition, the maximum systemic exposure achievable after dosing the conventional tablet is limited, such that at conventional tablet doses in excess of 150mg, there is a significant reduction in bicalutamide bioavailability. At conventional tablet doses above 300mg, no further significant increase in systemic exposure is achievable

It would be desirable to extend the therapeutic potential of 4'-cyano-α',α',α'-trifluoro-3-(4-fluorophenylsulphonyl)-2-hydroxy-2-methylpropiono-*m*-toluidide by increasing the bioavailability of the drug and/or reducing inter-patient variability in plasma concentrations of the drug as a result of reduced inter-patient variability in the absorption of the drug.

Such increased bioavailability could be useful in enabling a reduction in the daily dose of 4'-cyano-α',α',α'-trifluoro-3-(4-fluorophenylsulphonyl)-2-hydroxy-2-methylpropiono-*m-*toluidide required to achieve the same level of bioavailability seen with a conventional formulation.

A possible benefit of achieving relatively higher bioavailability could also be the ability to extend treatment to more advanced stages of prostate cancer than are currently treated with the conventional formulations. This could be useful, for example, for treating patients with metastatic prostate cancer, using for example 4'-cyano-α',α',α'-trifluoro-3-(4-fluorophenylsulphonyl)-2-hydroxy-2-methylpropiono-*m*-toluidide as a monotherapy (ie, not in combination with LHRH analogue therapy or surgical castration).

As another advantage, it would also be desirable to reduce inter-patient variability in plasma concentrations of 4'-cyano-α',α',α'-trifluoro-3-(4-fluorophenylsulphonyl)-2-hydroxy-2-methylpropiono-*m*-toluidide as a result of reduced inter-patient variability in the absorption of the drug. This would increase predictability of the treatment and increase uniformity of treatment in a patient population.

It would also be desirable to provide a 4'-cyano-α',α',α'-trifluoro-3-(4-fluorophenylsulphonyl)-2-hydroxy-2-methylpropiono-*m*-toluidide formulation having good storage stability.

EP-0988863 deals with the issue of increasing the bioavailability of poorly soluble drugs in general. 4'-cyano-α',α',α'-trifluoro-3-(4-fluorophenylsulphonyl)-2-hydroxy-2-methylpropiono-*m*-toluidide is not specifically addressed. The disclosed solution is to provide a formulation comprising a water-insoluble complex of the drug and a water-insoluble ionic polymer. No specific class of polymer is required, and the polymer can be cationic or anionic, but must have a molecular weight greater than about 80,000 D and a glass transition temperature equal or greater than about 50°C.

EP-1027886 also deals with the issue of increasing the bioavailability of poorly soluble drugs in general. Again, 4'-cyano-α',α',α'-trifluoro-3-(4-fluorophenylsulphonyl)-2-hydroxy-2-methylpropiono-*m*-toluidide is not specifically addressed. The disclosed solution is to provide a solid dispersion formulation comprising a low-solubility drug and a polymer. The latter can be one of many possible polymers, as long as it has a glass transition temperature of at least 100°C measured at 50% relative humidity.

The present invention aims to improve upon the conventional formulation of bicalutamide (racemic 4'-cyano-α',α',α'-trifluoro-3-(4-fluorophenylsulphonyl)-2-hydroxy-2-methylpropiono-*m*-toluidide) by increasing the therapeutic potential of 4'-cyano-α',α',α'-trifluoro-3-(4-fluorophenylsulphonyl)-2-hydroxy-2-methylpropiono-*m*-toluidide as discussed above.

The present invention aims to provide a 4'-cyano-α',α',α'-trifluoro-3-(4-fluorophenylsulphonyl)-2-hydroxy-2-methylpropiono-*m*-toluidide formulation having enhanced storage stability.

### SUMMARY OF THE INVENTION

The present invention fulfils at least one of these aims by providing a pharmaceutical formulation for mucosal administration to a patient, the formulation comprising 4'-cyano-α',α',α'-trifluoro-3-(4-fluorophenylsulphonyl)-2-hydroxy-2-methylpropiono-*m*-toluidide in a solid dispersion with PVP. In one embodiment, >50% of the 4'-cyano-α',α',α'-trifluoro-3-(4-fluorophenylsulphonyl)-2-hydroxy-2-methylpropiono-*m*-toluidide is provided in the form of the R-enantiomer.

The invention also provides a daily pharmaceutical dose of 4'-cyano-α',α',α'-trifluoro-3-(4-fluorophenylsulphonyl)-2-hydroxy-2-methylpropiono-*m*-toluidide mucosally administrable to a patient for treating and/or reducing the risk of prostate cancer in the patient, wherein the dose comprises 10 to 1500mg of 4'-cyano-α',α',α'-trifluoro-3-(4-fluorophenylsulphonyl)-2-hydroxy-2-methylpropiono-*m*-toluidide in a solid dispersion with PVP. In one embodiment, >50% of the 4'-cyano-α',α',α'-trifluoro-3-(4-fluorophenylsulphonyl)-2-hydroxy-2-methylpropiono-*m*-toluidide is provided in the form of the R-enantiomer. In a further embodiment the dose comprises 25 to 600mg of 4'-cyano-α',α',α'-trifluoro-3-(4-fluorophenylsulphonyl)-2-hydroxy-2-methylpropiono-*m*-toluidide.

Further aspects of the invention relate to the use of PVP in solid dispersion with 4'-cyano-α',α',α'-trifluoro-3-(4-fluorophenylsulphonyl)-2-hydroxy-2-methylpropiono-*m-*toluidide (in one embodiment wherein >50% of the 4'-cyano-α',α',α'-trifluoro-3-(4-fluorophenylsulphonyl)-2-hydroxy-2-methylpropiono-*m*-toluidide is provided in the form of the R-enantiomer), in the manufacture of a medicament mucosally administrable to a patient, for
(a) increasing the bioavailability of 4'-cyano-α',α',α'-trifluoro-3-(4-fluorophenylsulphonyl)-2-hydroxy-2-methylpropiono-*m*-toluidide in the patient; or
(b) treating and/or reducing the risk of prostate cancer in the patient. As explained below, reducing the risk of prostate cancer includes reducing the risk of re-occurrence of prostate cancer.

In addition, the invention relates to the use of PVP in solid dispersion with 4'-cyano-α',α',α'-trifluoro-3-(4-fluorophenylsulphonyl)-2-hydroxy-2-methylpropiono-*m*-toluidide in the manufacture of a medicament mucosally administrable to patients, for reducing inter-patient variability in plasma concentrations of 4'-cyano-α',α',α'-trifluoro-3-(4-fluorophenylsulphonyl)-2-hydroxy-2-methylpropiono-*m*-toluidide. In one embodiment, >50% of the 4'-cyano-α',α',α'-trifluoro-3-(4-fluorophenylsulphonyl)-2-hydroxy-2-methylpropiono-*m*-toluidide is provided in the form of the R-enantiomer.

Furthermore, the invention relates to the use of PVP in solid dispersion with 4'-cyano-α',α',α'-trifluoro-3-(4-fluorophenylsulphonyl)-2-hydroxy-2-methylpropiono-*m*-toluidide in the manufacture of a pharmaceutical formulation, for enhancing the storage stability of the 4'-cyano-α',α',α'-trifluoro-3-(4-fluorophenylsulphonyl)-2-hydroxy-2-methylpropiono-*m-*toluidide in the formulation. In one embodiment, >50% of the 4'-cyano-α',α',α'-trifluoro-3-(4-fluorophenylsulphonyl)-2-hydroxy-2-methylpropiono-*m*-toluidide is provided in the form of the R-enantiomer.

Another aspect of the invention relates to the use of PVP in solid dispersion with 4'-cyano-α',α',α'-trifluoro-3-(4-fluorophenylsulphonyl)-2-hydroxy-2-methylpropiono-*m-*toluidide (in one embodiment wherein >50% of the 4'-cyano-α',α',α'-trifluoro-3-(4-fluorophenylsulphonyl)-2-hydroxy-2-methylpropiono-*m*-toluidide is provided in the form of the R-enantiomer), in the manufacture of a pharmaceutical formulation mucosally administrable to a patient, for enhancing the storage stability of the 4'-cyano-α',α',α'-trifluoro-3-(4-fluorophenylsulphonyl)-2-hydroxy-2-methylpropiono-*m*-toluidide in the formulation in addition to increasing the bioavailability of 4'-cyano-α',α',α'-trifluoro-3-(4-fluorophenylsulphonyl)-2-hydroxy-2-methylpropiono-*m*-toluidide in the patient and/or reducing inter-patient variability in plasma concentrations of 4'-cyano-α',α',α'-trifluoro-3-(4-fluorophenylsulphonyl)-2-hydroxy-2-methylpropiono-*m*-toluidide.

As explained further below, an additional advantage of the present invention for solid dispersions with relatively high drug loads is an improvement in drug dissolution compared with similar solid dispersions where a higher proportion of the drug is provided in the S-form.

### FIGURES

Fig. 1 Dissolution of bicalutamide (ie, racemic 4'-cyano-α',α',α'-trifluoro-3-(4-fluorophenylsulphonyl)-2-hydroxy-2-methylpropiono-*m*-toluidide) from various solid dispersion formulations (50mg bicalutamide in 900ml of media).

**Key:-**

| | |
|---|---|
| Diamonds | - conventional bicalutamide tablet formulation |
| Triangles | - PEG4000 |
| Rectangles | - PVP |
| Crosses | - PLA:PEG [2kDa:2kDa] |

Fig. 2 Dissolution of bicalutamide from solid dispersion formulations (50mg bicalutamide in 900ml of media) with or without SDS.

**Key:-**

| | |
|---|---|
| Circles | - with 5% SDS |
| Squares | - without SDS |

Fig. 3 Dissolution of bicalutamide and optically pure R-4'-cyano-α',α',α'-trifluoro-3-(4-fluorophenylsulphonyl)-2-hydroxy-2-methylpropiono-*m*-toluidide from solid dispersion formulations (50mg 4'-cyano-α',α',α'-trifluoro-3-(4-fluorophenylsulphonyl)-2-hydroxy-2-methylpropiono-*m*-toluidide in 900ml of media, 1:3 drug : PVP ratio).

**Key:-**

| | |
|---|---|
| Diamonds | - bicalutamide |
| Squares | - R-4'-cyano-α',α',α'-trifluoro-3-(4-fluorophenylsulphonyl)-2-hydroxy-2-methylpropiono-*m*-toluidide |
| Triangles | - conventional bicalutamide tablet formulation |

Fig. 4 Dissolution of bicalutamide and pure R-4'-cyano-α',α',α'-trifluoro-3-(4-fluorophenylsulphonyl)-2-hydroxy-2-methylpropiono-*m*-toluidide from solid dispersion formulations (50mg 4'-cyano-α',α',α'-trifluoro-3-(4-fluorophenylsulphonyl)-2-hydroxy-2-methylpropiono-*m*-toluidide in 900ml of media, 1:1 drug: PVP ratio).

**Key:-**

| | |
|---|---|
| Diamonds | - bicalutamide |
| Squares | - R-4'-cyano-α',α',α'-trifluoro-3-(4-fluorophenylsulphonyl)-2-hydroxy-2-methylpropiono-*m*-toluidide |
| Triangles | - conventional bicalutamide tablet formulation |

### DETAILED DESCRIPTION OF THE INVENTION

The inventors chose to investigate solid dispersion formulations as a possible means of fulfilling at least one of the aims stated above. In terms of the aim of increasing the therapeutic potential of 4'-cyano-α',α',α'-trifluoro-3-(4-fluorophenylsulphonyl)-2-hydroxy-2-methylpropiono-*m*-toluidide, the inventors sought to increase the therapeutic potential by achieving one or both of an increase the bioavailability of 4'-cyano-α',α',α'-trifluoro-3-(4-fluorophenylsulphonyl)-2-hydroxy-2-methylpropiono-*m*-toluidide and a decrease in inter-patient variability in plasma concentrations of 4'-cyano-α',α',α'-trifluoro-3-(4-fluorophenylsulphonyl)-2-hydroxy-2-methylpropiono-*m*-toluidide.

The prior art teaches a very wide range of possible polymers for solid dispersion, in order to increase the bioavailability of drugs in general. The inventors have now surprisingly found that the therapeutic potential of 4'-cyano-α',α',α'-trifluoro-3-(4-fluorophenylsulphonyl)-2-hydroxy-2-methylpropiono-*m*-toluidide can be increased by formulating 4'-cyano-α',α',α'-trifluoro-3-(4-fluorophenylsulphonyl)-2-hydroxy-2-methylpropiono-*m*-toluidide in a solid dispersion specifically with PVP. In one embodiment, >50% of the 4'-cyano-α',α',α'-trifluoro-3-(4-fluorophenylsulphonyl)-2-hydroxy-2-methylpropiono-*m*-toluidide is provided in the form of the R-enantiomer. As the non-limiting example section below demonstrates, such an increase in therapeutic potential for 4'-cyano-α',α',α'-trifluoro-3-(4-fluorophenylsulphonyl)-2-hydroxy-2-methylpropiono-*m*-toluidide is not achieved with other polymers.

PVP is also known by various other names, such as polyvinylpyrrolidone, poly[1-(2-oxo-1-pyrrolidinyl)ethylene], polyvidone and 1-vinyl-2-pyrrolidinone polymer. PVP is available in various grades as shown in the following table.

| K-value | Approximate Molecular Weight |
|---|---|
| 12 | 2500 |
| 15 | 8000 |
| 17 | 10 000 |
| 25 | 30 000 |
| 30 | 50 000 |
| 60 | 400 000 |
| 90 | 1 000 000 |
| 120 | 3 000 000 |

In one embodiment, the present invention uses PVP having a K-value ≤90. For example, the PVP has a K-value range ≤60, or ≤30, but ≥15, ≥17 or ≥25. In one example, the K-value is selected from 25, 17, 15 and 12.

In one embodiment, the 4'-cyano-α',α',α'-trifluoro-3-(4-fluorophenylsulphonyl)-2-hydroxy-2-methylpropiono-*m*-toluidide is in a solid dispersion with at least one PVP polymer. Thus, it is contemplated that a mixture of two or more PVP polymers differing in K-values can be used.

A preferred ratio of 4'-cyano-α',α',α'-trifluoro-3-(4-fluorophenylsulphonyl)-2-hydroxy-2-methylpropiono-*m*-toluidide: PVP by weight is from 1:0.25 to 1:10. More preferably the lower limit of this range is 1:1, 1:2, 1:3 or 1:>3. Preferably, the upper limit of this range is 1:≤3, 1:5 or 1:7. Particularly preferred ratios are 1:5, 1:4 and 1:3. In one embodiment, the range is 1:>3 to 1:10. In another embodiment, the range is 1:0.25 to 1:≤3 and the solid dispersion includes a wetting agent. Further discussion of wetting agents appears below.

One aspect of the invention provides a daily pharmaceutical dose of 4'-cyano-α',α',α'-trifluoro-3-(4-fluorophenylsulphonyl)-2-hydroxy-2-methylpropiono-*m*-toluidide mucosally administrable to a patient for treating and/or reducing the risk of prostate cancer in the patient, wherein the dose comprises 10 to 1500mg of 4'-cyano-α',α',α'-trifluoro-3-(4-fluorophenylsulphonyl)-2-hydroxy-2-methylpropiono-*m*-toluidide in a solid dispersion with PVP. In one embodiment the dose comprises 25 to 600mg of 4'-cyano-α',α',α'-trifluoro-3-(4-fluorophenylsulphonyl)-2-hydroxy-2-methylpropiono-*m*-toluidide.

Another aspect of the invention provides a daily pharmaceutical dose of 4'-cyano-α',α',α'-trifluoro-3-(4-fluorophenylsulphonyl)-2-hydroxy-2-methylpropiono-*m*-toluidide mucosally administrable to a patient for treating and/or reducing the risk of prostate cancer in the patient, wherein the dose comprises 10 to 1500mg of 4'-cyano-α',α',α'-trifluoro-3-(4-fluorophenylsulphonyl)-2-hydroxy-2-methylpropiono-*m*-toluidide in a solid dispersion with PVP and >50% of the 4'-cyano-α',α',α'-trifluoro-3-(4-fluorophenylsulphonyl)-2-hydroxy-2-methylpropiono-*m*-toluidide is provided in the form of the R-enantiomer. In one embodiment the dose comprises 25 to 600mg of 4'-cyano-α',α',α'-trifluoro-3-(4-fluorophenylsulphonyl)-2-hydroxy-2-methylpropiono-*m*-toluidide.

Preferably, the dose comprises 1500, 1250, 1000, 800, 700, 600, 500, 450, 400, 300, 200, 150, 125, 100, 75, 50mg, 25, 15 or 10mg of 4'-cyano-α',α',α'-trifluoro-3-(4-fluorophenylsulphonyl)-2-hydroxy-2-methylpropiono-*m*-toluidide. In one example, the dose comprises 150 or 450mg of 4'-cyano-α',α',α'-trifluoro-3-(4-fluorophenylsulphonyl)-2-hydroxy-2-methylpropiono-*m*-toluidide.

In most countries, the conventional once a day dose of Casodex™ is 50mg (Casodex50), when used in combination with LHRHa for the treatment of metastatic prostatic cancer or 150mg (Casodex150), when used as monotherapy in the treatment of locally advanced prostate cancer. With an average half-life of about seven days, it generally takes between one and two months before a patient attains the optimal steady state concentration of the drug in the blood/plasma, which is on average about ten times higher than that after the initial dose. It is believed that all or predominantly all of the activity of Casodex is due to the R-enantiomer form of 4'-cyano-α',α',α'-trifluoro-3-(4-fluorophenylsulphonyl)-2-hydroxy-2-methylpropiono-*m*-toluidide. Accordingly, to attain a pharmacokinetic bioequivalent dose to the conventional 50mg dose of Casodex with a formulation comprising only the R-form may only require a 25mg dose, furthermore, with the enhanced bioavailability offered by the novel formulation comprising PVP the dose could be as low as 10 mg. As noted above, it may take a month or more to reach optimal steady state levels of the drug with the conventional once a day 50 mg or 150 mg Casodex treatment. With the novel formulation of the invention, it may prove possible and beneficial to reduce the time to reach this steady state drug level in the blood by administering to the patient one or more loading doses of Casodex. As the average steady state dose concentration is about 10 times that after a single dose, such a loading dose could be up to 500mg (10x50mg) for combination therapy or 1500mg (10 x 150mg) for monotherapy. Reducing the time taken to reach the optimal steady state dose concentration may provide clinical benefit.

For example, the loading dose required to achieve steady state concentration of the drug in the blood equivalent to Casodex150 will depend upon the relative bioavailability of the novel formulation at doses > 150mg to that at a dose which is bioequivalent (BE) to Casodex150. Assuming dose linearity over a very wide range, the number of loading doses at a multiple of the BE dose is estimated to be:
Loading dose 3x the BE dose: steady state achieved after about 5 doses.
Loading dose 4x the BE dose: steady state achieved after about 3 doses.
Loading dose 6x the BE dose: steady state achieved after about 2 doses.
Loading dose 10x the BE dose: steady state achieved after about 1 dose.

By maintenance dose we mean a dose that is approximately (+/- ca25%) bioequivalent with conventional Casodex (i.e. Casodex50 or Casodex 150, or doses therebetween). By loading dose we mean a dose that is at least 2-fold greater than the maintenance dose. In separate examples the loading dose could be 3-fold, 4-fold, 5-fold, 6-fold, 8-fold, or 10-fold the maintenance dose. The number of daily loading doses required to be administered prior to switching to the maintenance dose will depend on the concentration achieved by the loading dose(s). This number can easily be determined by the person skilled in the art.

By way of example with conventional Casodex50, a patient might be given a single loading dose of the novel formulated drug according to the present invention that, assuming dose linearity, is bioequivalent to about 500mg or 600mg of conventional Casodex, followed by successive daily doses of conventional Casodex50, or a BE dose of the novel formulation comprising 4'-cyano-α',α',α'-trifluoro-3-(4-fluorophenylsulphonyl)-2-hydroxy-2-methylpropiono-*m*-toluidide in a solid dispersion with PVP. In another example, the patient might be given two successive daily loading doses of the novel formulated drug according to the present invention that is say bioequivalent to a 300mg dose of conventional Casodex, followed by successive daily doses of conventional Casodex50, or a BE dose of the novel formulation comprising 4'-cyano-α',α',α'-trifluoro-3-(4-fluorophenylsulphonyl)-2-hydroxy-2-methylpropiono-*m*-toluidide in a solid dispersion with PVP.

Additional excipients may be included in the formulation or dose. For example, the formulation or dose may comprise one or more fillers, binders, disintegrants and/or lubricants.

Suitable fillers include, for example, lactose, sugar, starches, modified starches, mannitol, sorbitol, inorganic salts, cellulose derivatives (e.g. microcrystalline cellulose, cellulose), calcium sulphate, xylitol and lactitol.

Suitable binders include, for example, lactose, starches, modified starches, sugars, gum acacia, gum tragacanth, guar gum, pectin, wax binders, microcrystalline cellulose, methylcellulose, carboxymethylcellulose, hydroxypropyl methylcellulose, hydroxyethyl cellulose, hydroxypropyl cellulose, copolyvidone, gelatin and sodium alginate.

Suitable disintegrants include, for example, crosscarmellose sodium, crospovidone, polyvinylpyrrolidone, sodium starch glycollate, corn starch, microcrystalline cellulose, hydroxypropyl methylcellulose and hydroxypropyl cellulose. Suitable lubricants include, for example, magnesium stearate, stearic acid, palmitic acid, calcium stearate, talc, carnuba wax, hydrogenated vegetable oils, mineral oil, polyethylene glycols and sodium stearyl fumarate.

Additional conventional excipients which may be added include preservatives, stabilisers, anti-oxidants, silica flow conditioners, antiadherents or glidants.

Other suitable fillers, binders, disintegrants, lubricants and additional excipients which may be used are described in the Handbook of Pharmaceutical Excipients, 3rd Edition; The Theory and Practice of Industrial Pharmacy, 3rd Edition 1956; Pharmaceutical Dosage Forms 1998; Modem Pharmaceutics, 3rd Edition 1995; Remington's Pharmaceutical Sciences 20th Edition 2000.

Preferably, the 4'-cyano-α',α',α'-trifluoro-3-(4-fluorophenylsulphonyl)-2-hydroxy-2-methylpropiono-*m*-toluidide will be present in an amount of 1 to 80%, and preferably from 1 to 50% (more preferably 2 to 20% or 2 to 15%) by weight of the solid dispersion.

Preferably, one or more fillers will be present in an amount of 1 to 70% by weight of the formulation or dose.

Preferably, one or more binders will be present in an amount of 2 to 40% by weight of the formulation or dose.

Preferably, one or more disintegrants will be present in an amount of 1 to 10%, and especially 4 to 6% by weight of the formulation or dose.

It will be appreciated that a particular excipient may act as both a binder and a filler, or as a binder, a filler and a disintegrant. Typically the combined amount of filler, binder and disintegrant comprises, for example, 1 to 90% by weight of the formulation or dose.

Preferably, one or more lubricants will be present in an amount of 0.5 to 3%, and especially 1 to 2% by weight of the formulation or dose.

Preferably, one or more wetting agents will be present in the solid dispersion in an amount of 0.1 to 5% (eg, 1 to 2%) by weight of the solid dispersion. The presence of a wetting agent provides a further enhancement of the increase in therapeutic potential achieved with the present invention. Examples of suitable wetting agents include sodium dodecyl sulphate (sodium lauryl sulphate); docusate sodium; polyoxyethylen sorbitan fatty acid esters, eg polysorbates 20, 40, 60 and 80; polyoxyethylene castor oil derivatives, eg Cremophor RH40™; and poloxamers.

Methods for preparing solid dispersions are known in the art and typically comprise the steps of dissolving the drug and the polymer in a common solvent and evaporating the solvent. The solvent can be routinely selected according to the polymer used. Examples of solvents are: acetone, acetone/dichloromethane, methanol/dichloromethane, acetone/water, acetone/ethanol, dichloromethane/ethanol or ethanol/water. Methods for evaporating solvent include rotary evaporation, spray drying, lyophilisation and thin film evaporation. Other techniques may be used such as melt extrusion, solvent controlled precipitation, pH controlled precipitation and supercritical fluid technology.

When referring to a solid dispersion we do not exclude the possibility that a proportion of the 4'-cyano-α',α',α'-trifluoro-3-(4-fluorophenylsulphonyl)-2-hydroxy-2-methylpropiono-*m*-toluidide may be dissolved within the PVP, the exact proportion, if any, will depend upon the particular PVP polymer selected.

In the formulations of the invention, at least some of the 4'-cyano-α',α',α'-trifluoro-3-(4-fluorophenylsulphonyl)-2-hydroxy-2-methylpropiono-*m*-toluidide may be present in amorphous form in the solid dispersion with the PVP. The provision of the 4'-cyano-α',α',α'-trifluoro-3-(4- fluorophenylsulphonyl)-2-hydroxy-2-methylpropiono-*m*-toluidide in amorphous form is additionally advantageous, since it further increases the solubility and dissolution rate of the 4-cyano-α',α',α'-trifluoro-3-(4-fluorophenylsulphonyl)-2-hydroxy-2-methylpropiono-*m*-toluidide, thereby enhancing the increase in therapeutic potential achieved with the present invention. Whether or not drug is present in amorphous form can be determined by conventional thermal analysis or X-ray diffraction. In one embodiment, at least 25% of the 4'-cyano-α',α',α'-trifluoro-3-(4-fluorophenylsulphonyl)-2-hydroxy-2-methylpropiono-*m*-toluidide in the formulation is present in amorphous form. More preferably, this amount is at least 20%, 30%, 40%, 50%, 75%, 90%, 95% or 99%. The most preferred embodiment is where 100% of the 4'-cyano-α',α',α'-trifluoro-3-(4-fluorophenylsulphonyl)-2-hydroxy-2-methylpropiono-*m*-toluidide in the formulation is in amorphous form.

The formulations and doses are mucosally administrable, ie administrable to mucosal membranes for absorption across the membranes. To this end, suitable routes of administration include administration by inhalation, as well as oral, intranasal and rectal administration. Oral administration is particularly preferred. A tablet or other form of the formulation would be chosen by the skilled addressee according to the route of administration.

The 4'-cyano-α',α',α'-trifluoro-3-(4-fluorophenylsulphonyl)-2-hydroxy-2-methylpropiono-*m*-toluidide is useful to provide an anti-androgenic effect, in that this compound blocks androgen activity in a patient. The anti-androgenic effect is useful for treating cancer, for example prostate cancer. Particular examples are advanced prostate cancer and early prostate cancer. The anti-androgenic effect may be useful for prophylaxis, in order to reduce the risk of prostate cancer occurrence in patients or re-occurrence (eg, following prostatectomy or radiation therapy aimed at curing the patient). This could be especially useful in men genetically pre-disposed to prostate cancer. Conventional methods are available to classify patients according to their risk of contracting prostate cancer, for example by assessment of family history and measurements over time of particular blood proteins such as prostate specific antigen (PSA). Other uses for the anti-androgenic effect are the treatment of a non-malignant disease of the prostate gland (eg, benign prostatic hyperplasia or hypertrophy) and acne.

The patient can be a human male, eg an adult, but the treatment of other mammals is also contemplated.

In one embodiment of the formulation or dose, ≥50%, ≥60%, ≥65%, ≥70%, ≥80%, ≥85%, ≥90%, ≥95%, ≥98% or ≥99% or thereabout of the 4'-cyano-α',α',α'-trifluoro-3-(4-fluorophenylsulphonyl)-2-hydroxy-2-methylpropiono-*m*-toluidide is provided in the form of the R-enantiomer. In a preferred embodiment, 100% or substantially 100% of the 4'-cyano-α',α',α'-trifluoro-3-(4-fluorophenylsulphonyl)-2-hydroxy-2-methylpropiono-*m*-toluidide is provided in the form of the R-enantiomer. By "substantially 100%" we mean that the 4'-cyano-α',α',α'-trifluoro-3-(4-fluorophenylsulphonyl)-2-hydroxy-2-methylpropiono-*m-*toluidide is provided as the pure R-enantiomer, or there is a trace (<1 %) of the S-enantiomer present. As the experimental section below shows, the predominance of the R-enantiomer in the present invention provides for a 4'-cyano-α',α',α'-trifluoro-3-(4-fluorophenylsulphonyl)-2-hydroxy-2-methylpropiono-*m*-toluidide formulation with good storage stability and an enhanced therapeutic potential.

### EXPERIMENTAL

### A: Comparative Examples

The following examples are not according to the present invention, but are included to provide a suitable context for the interpretation of the examples according to the present invention (see section B).

### In Vitro Assessment of Various Solid Dispersion Formulations

The inventors prepared a formulation of a solid dispersion of bicalutamide (racemic 4'-cyano-α',α',α'-trifluoro-3-(4-fluorophenylsulphonyl)-2-hydroxy-2-methylpropiono-*m-*toluidide) with a representative PVP polymer (in this case PVP K-25) and compared this against solid dispersions using several different polymers with bicalutamide. A conventional bicalutamide tablet formulation was also included for comparison. The formulations were assessed for an improvement in therapeutic potential using an *in vitro* dissolution test.

Each formulation had a weight ratio of bicalutamide:polymer of 1:5. The following polymers were used to produce solid dispersions:- polyethylene glycol (PEG) 4000, PLA:PEG [2kDa, 2kDa] (a di-block copolymer of poly(lactide):polyethylene glycol) and PVP K-25.

### Preparation of Solid Dispersion Formulations

Solid dispersions having a 1:5 ratio by weight of bicalutamide:polymer were prepared as follows.

0.5g of bicalutamide and 2.5g of polymer were weighed directly into a 250ml round bottom flask and dissolved in 80ml of acetone:dichloromethane (3:1). The solvent was removed on a rotary evaporator or by spray drying. The formulation was placed in a vacuum oven and dried under high vacuum at 40°C for 24 hours.

The formulation was retrieved from the flask and dry milled using a Fritsch mill. The formulation was then dried for a further 24 hours under high vacuum at 40°C.

In order to produce formulations having ratios other than 1:5, weights and volumes in the process should be adjusted so that they are pro-rata to those described above.

### In vitro Dissolution Test

### (a) PVP Solid Dispersion v. Solid Dispersion With Other Polymers

The formulations were weighed into hard gelatin capsules (equivalent to 50mg drug) and dissoluted in 900ml media [0.25% sodium dodecyl sulphate solution] for one hour at 37°C (paddle speed 75rpm). 5ml samples were then removed with a plastic syringe at 5, 10, 20, 30, 45 and 60 minutes. Each sample was centrifuged (14,000rpm) at ambient temperature for 15 minutes and then analysed by HPLC using the following conditions:-

| | |
|---|---|
| Eluent: | 58% ACN/42% water/0.2% formic acid |
| Column: | 15cm Luna 5um, 3mm id column (with guard) |
| Detection wavelength: | 270nm |
| Flow rate: | 1ml/min |
| Temperature: | ambient |
| Injection: | 10ul |
| Retention time: | approximately 2 minutes |

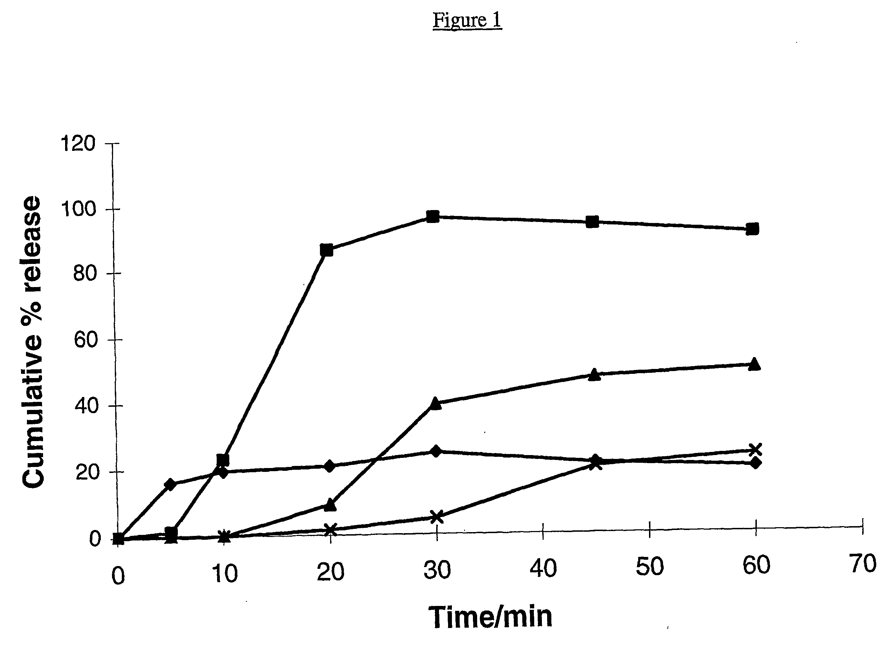

Figure 1 shows the results of *in vitro* dissolution tests performed on the various solid dispersions. As Fig.1 shows, 100% of bicalutamide in solution was achieved with the PVP solid dispersion and supersaturation was maintained over the 60 minute test (ie, no drug precipitation was observed). Compare this against the results for the PLA:PEG solid dispersion, which did not show any improvement over the conventional tablet formulation. The PEG4000 solid dispersion also was much inferior to the PVP formulation, the former achieving only approximately 50% of bicalutamide in solution for a 1:5 ratio.

### Enhancement of Therapeutic Potential Using A Wetting Agent

Solid dispersions were prepared using a spray drying method for solvent removal with and without 5% sodium dodecyl sulphate (SDS) as a wetting agent. The solid dispersions had a 1:3 ratio by weight of bicalutamide:polymer.

### Preparation of Solid Dispersions

### (a) without SDS

3.0g of drug (bicalutamide) and 9.0g of PVP K-25 polymer were added to 400ml of the solvent mixture, Acetone/Dichloromethane (3:1). Complete dissolution was achieved with stirring. The solution was delivered to the spray dryer and the solvent removed. The product was a free flowing white powder.

### (b) with 5% SDS

0.6g of SDS was added to 400ml of the solvent mixture, Acetone/Dichloromethane (3:1). Complete dissolution was achieved by stirring and heat. The solution was cooled before the addition of 3.0g of drug (bicalutamide) and 9.0g of PVP K-25 polymer complete dissolution was achieved with stirring. The solution was delivered to the spray dryer and the solvent removed. The product was a free flowing white powder.

### In vitro Dissolution Test

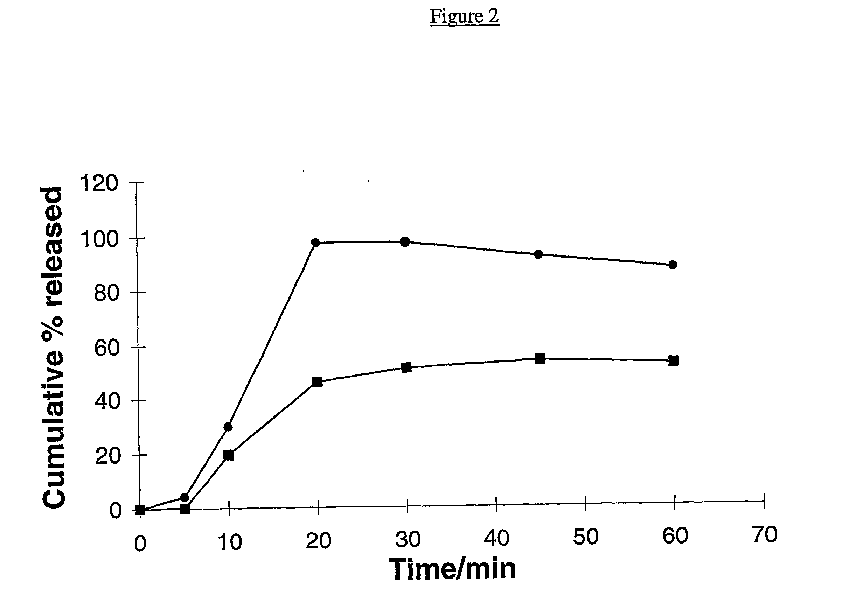

The test was performed following the protocol above. Figure 2 shows a comparison of cumulative % bicalutamide released v. time for the two formulations. As Fig. 1 shows, for solid dispersions having a 1:3 ratio by weight of bicalutamide:polymer. the formulation including SDS displayed enhanced bicalutamide release compared to the formulation that did not include SDS.

### B: Examples in accordance with the invention

### Enhancement of therapeutic potential provided by the R-enantiomer

### (i) At a 1:3 ratio

A solid dispersion was made that had a 1:3 ratio by weight of R-4'-cyano-α',α',α'-trifluoro-3-(4-fluorophenylsulphonyl)-2-hydroxy-2-methylpropiono-*m*-toluidide (100% of the R-enantiomer): PVP K-25 polymer. Production was by a spray drying method for solvent removal with 5% sodium dodecyl sulphate (SDS) as a wetting agent. A second solid dispersion was also made by a spray drying method with 5% SDS, but this solid dispersion had a 1:3 ratio by weight of bicalutamide (ie, racemic R-4'-cyano-α',α',α'-trifluoro-3-(4-fluorophenylsulphonyl)-2-hydroxy-2-methylpropiono-*m*-toluidide) : PVP K-25 polymer.

### In vitro Dissolution Test

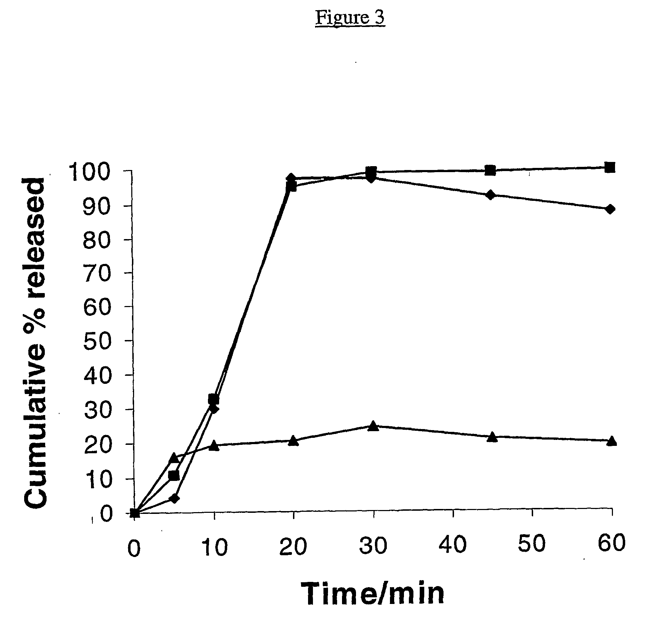

The test was performed following the protocol above. Figure 3 shows a comparison of cumulative % 4'-cyano-α',α',α'-trifluoro-3-(4-fluorophenylsulphonyl)-2-hydroxy-2-methylpropiono-*m*-toluidide released v. time for the two formulations and for a conventional 50mg bicalutamide tablet formulation. As Fig. 3 shows, the solid dispersion according to the invention, which had 100% of the R-enantiomer, displayed enhanced drug release compared to the conventional formulation. The enhancement was similar to that achieved by the bicalutamide solid dispersion.

### (ii) At a 1:1 ratio

The protocol in part (i) was followed, with the exception that the drug : PVP ratio for both formulations was changed to 1:1.

### In vitro Dissolution Test

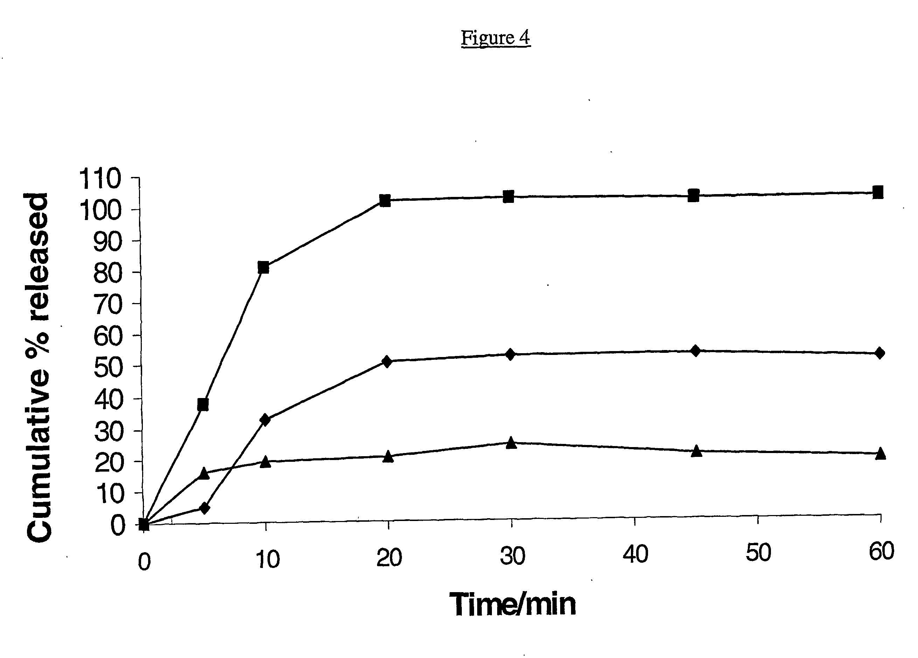

The test was performed following the protocol above. Figure 4 shows a comparison of cumulative % 4'-cyano-α',α',α'-trifluoro-3-(4-fluorophenylsulphonyl)-2-hydroxy-2-methylpropiono-*m*-toluidide released v. time for the two formulations with a 1:1 ratio. Again, both solid dispersion formulations displayed enhanced drug release compared to the conventional formulation. Note, however, that at the 1:1 ratio the formulation according to the present invention displayed a release profile that was enhanced when compared with the bicalutamide solid dispersion. Indeed, the formulation according to the invention achieved 100% of drug in solution and supersaturation was maintained over the 60 minute test (ie, no drug precipitation was observed).

Thus, provision of the drug (4'-cyano-α',α',α'-trifluoro-3-(4-fluorophenylsulphonyl)-2-hydroxy-2-methylpropiono-*m*-toluidide) in the R-enantiomer form according to the invention, particularly where 100% of the drug is in the R-form, provides an additional drug dissolution advantage. Provision of the R-enantiomer generally gives a higher percentage drug dissolution (eg, determined as % drug dissolved after 60 minutes in the *in vitro* dissolution test described above) than another solid dispersion that is identical but for the provision of a significantly higher proportion of the drug in the S-form (but where the total proportion of drug : PVP remains the same). The advantage is most evident as one increases the total proportion of drug : PVP in the formulation (eg, for proportions of 1:0.25 to 1:<3). Thus, for a 1:1 drug:PVP ratio, one finds a higher percentage dissolution of drug after 60 minutes in the *in vitro* dissolution test with a solid dispersion comprising 100% of the R-enantiomer as compared to a solid dispersion containing the racemate. The advantage is also expected to be achieved with other formulations of the invention wherein > 50% of the drug is provided in the R-form.

### Enhancement of storage stability provided by the R-enantiomer

Solid dispersion formulations were prepared as in part B(i) above (ie, having a 1:3 ratio of drug : PVP).

The storage stability of the formulations was assessed using X-ray diffraction (XRD) as follows. The formulations were placed in sealed glass amber vials and stored at the following conditions, 4°C, 25°C/60%RH, 50°C and 40°C/75%RH (RH, relative humidity) for three months. After three months the samples were removed and analysed by XRD (X-ray diffraction) to determine the presence or absence of crystallinity. The results are presented in the following table.

| **Drug:Polymer** | **Storage** | **Three months** |
|---|---|---|
| **ratio** | **condition** | **XRD** |
| 1:3 | Initial | X |
| (R/S- drug) | 4°C | √ |
| | 25°C/60%RH | √ |
| | 50°C | √ |
| | 40°C/75%RH | √ |
| | | |
| 1:3 | Initial | X |
| (R- drug) | 4°C | X |
| | 25°C/60%RH | X |
| | 50°C | X |
| | 40°C/75%RH | X |

| | | |
|---|---|---|
| X = no crystallinity | | |
| √ = crystallinity | | |

As the results show, no crystallinity was detected after 3 months when the formulation according to the invention was stored under any of the conditions, indicating the superior stability of the formulation. With the bicalutamide (R/S-) formulation, however, the formulation was less stable under all of the experimental conditions, as indicated by the presence of crystallinity. The presence of crystallinity in the R/S sample corresponded with a reduction in the dissolution performance of the formulation when tested after 3 months of storage under all conditions.

## Claims

1. A pharmaceutical formulation for mucosal administration to a patient, the formulation comprising 4'-cyano-α',α',α'-trifluoro-3-(4-fluorophenylsulphonyl)-2-hydroxy-2-methylpropiono-*m*-toluidide in a solid dispersion with PVP.

2. A pharmaceutical formulation as claimed in claim 1, wherein >50% of the 4'-cyano-α',α',α'-trifluoro-3-(4-fluorophenylsulphonyl)-2-hydroxy-2-methylpropiono-*m*-toluidide is provided in the form of the R-enantiomer.

3. A daily pharmaceutical dose of 4'-cyano-α',α',α'-trifluoro-3-(4-fluorophenylsulphonyl)-2-hydroxy-2-methylpropiono-*m*-toluidide mucosally administrable to a patient for treating and/or reducing the risk of prostate cancer in the patient, wherein the dose comprises 10 to 1500mg of 4'-cyano-α',α',α'-trifluoro-3-(4-fluorophenylsulphonyl)-2-hydroxy-2-methylpropiono-*m*-toluidide in a solid dispersion with PVP.

4. A daily pharmaceutical dose of 4'-cyano-α',α',α'-trifluoro-3-(4-fluorophenylsulphonyl)-2-hydroxy-2-methylpropiono-*m*-toluidide as claimed in claim 3, wherein >50% of the 4'-cyano-α',α',α'-trifluoro-3-(4-fluorophenylsulphonyl)-2-hydroxy-2-methylpropiono-*m-*toluidide being provided in the form of the R-enantiomer.

5. The formulation of claim 1 or 2 or the dose of claim 3 or 4, wherein the ratio of 4'-cyano-α',α',α'-trifluoro-3-(4-fluorophenylsulphonyl)-2-hydroxy-2-methylpropiono-*m*-toluidide: PVP by weight is from 1:0.25 to 1:10.

6. The formulation or dose of claim 5, wherein the ratio of 4'-cyano-α',α',α'-trifluoro-3-(4-fluorophenylsulphonyl)-2-hydroxy-2-methylpropiono-*m*-toluidide: PVP by weight is 1:>3 to 1:10.

7. The formulation or dose of any preceding claim, wherein the solid dispersion includes a wetting agent.

8. The formulation or dose of claim 5, wherein the ratio of 4'-cyano-α',α',α'-trifluoro-3-(4-fluorophenylsulphonyl)-2-hydroxy-2-methylpropiono-*m*-toluidide: PVP by weight is 1:0.25 to 1:≤3 and the solid dispersion includes a wetting agent.

9. The formulation or dose of any preceding claim, wherein about ≥50%, ≥60%, ≥65%, ≥70%, ≥80%, ≥85%, ≥90%, ≥95%, ≥98% or ≥99% of the 4'-cyano-α',α',α'-trifluoro-3-(4-fluorophenylsulphonyl)-2-hydroxy-2-methylpropiono-*m*-toluidide is provided in the form of the R-enantiomer.

10. The formulation or dose of claim 9, wherein substantially 100% of the 4'-cyano-α',α',α'-trifluoro-3-(4-fluorophenylsulphonyl)-2-hydroxy-2-methylpropiono-*m*-toluidide is provided in the form of the R-enantiomer.

11. The formulation or dose of any preceding claim, wherein the PVP has a K-value ≤90.

12. The formulation or dose of claim 11, wherein the 4'-cyano-α',α',α'-trifluoro-3-(4-fluorophenylsulphonyl)-2-hydroxy-2-methylpropiono-*m*-toluidide is in solid dispersion with PVP K-25.

13. A solid dispersion of PVP with 4'-cyano-α',α',α'-trifluoro-3-(4-fluorophenylsulphonyl)-2-hydroxy-2-methylpropiono-*m*-toluidide for use as a medicament.

14. A solid dispersion as claimed in claim 13, wherein >50% of the 4'-cyano-α',α',α'-trifluoro-3-(4-fluorophenylsulphonyl)-2-hydroxy-2-methylpropiono-*m*-toluidide is provided in the form of the R-enantiomer.

15. The solid dispersion of claim 13 or claim 14, wherein the solid dispersion includes a wetting agent.

16. Use of PVP in solid dispersion with 4'-cyano-α',α',α'-trifluoro-3-(4-fluorophenylsulphonyl)-2-hydroxy-2-methylpropiono-*m*-toluidide in the manufacture of a medicament mucosally administrable to a patient, for increasing the bioavailability of 4'-cyano-α',α',α'-trifluoro-3-(4-fluorophenylsulphonyl)-2-hydroxy-2-methylpropiono-*m-*toluidide in the patient.

17. Use of PVP in solid dispersion with 4'-cyano-α',α',α'-trifluoro-3-(4-fluorophenylsulphonyl)-2-hydroxy-2-methylpropiono-*m*-toluidide in the manufacture of a medicament mucosally administrable to patients, for reducing inter-patient variability in plasma concentrations of 4'-cyano-α',α',α'-trifluoro-3-(4-fluorophenylsulphonyl)-2-hydroxy-2-methylpropiono-*m*-toluidide.

18. Use of PVP in solid dispersion with 4'-cyano-α',α',α'-trifluoro-3-(4-fluorophenylsulphonyl)-2-hydroxy-2-methylpropiono-*m*-toluidide in the manufacture of a pharmaceutical formulation mucosally administrable to a patient, for increasing the bioavailability of 4'-cyano-α',α',α'-trifiuoro-3-(4-fluorophenylsulphonyl)-2-hydroxy-2-methylpropiono-*m*-toluidide in the patient and/or reducing inter-patient variability in plasma concentrations of 4'-cyano-α',α',α'-trifluoro-3-(4-fluorophenylsulphonyl)-2-hydroxy-2-methylpropiono-*m*-toluidide.

19. Use of PVP in solid dispersion with 4'-cyano-α',α',α'-trifluoro-3-(4-fluorophenylsulphonyl)-2-hydroxy-2-methylpropiono-*m*-toluidide in the manufacture of a medicament mucosally administrable to a patient, for treating and/or reducing the risk of prostate cancer in the patient.

20. The use according to any of claims 16 - 19, wherein >50% of the 4'-cyano-α',α',α'-trifluoro-3-(4-fluorophenylsulphonyl)-2-hydroxy-2-methylpropiono-*m*-toluidide is provided in the form of the R-enantiomer.

21. Use of PVP in solid dispersion with 4'-cyano-α',α',α'-trifluoro-3-(4-fluorophenylsulphonyl)-2-hydroxy-2-methylpropiono-*m*-toluidide, wherein >50% of the 4'-cyano-α',α',α'-trifluoro-3-(4-fluorophenylsulphonyl)-2-hydroxy-2-methylpropiono-*m-*toluidide is provided in the form of the R-enantiomer, in the manufacture of a pharmaceutical formulation, for enhancing the storage stability of the 4'-cyano-α',α',α'-trifluoro-3-(4-fluorophenylsulphonyl)-2-hydroxy-2-methylpropiono-*m*-toluidide in the formulation.

22. The use according to claim 19, wherein the medicament is provided as a daily dose of 4'-cyano-α',α',α'-trifluoro-3-(4-fluorophenylsulphonyl)-2-hydroxy-2-methylpropiono-*m-*toluidide and comprises 10 to 1500mg of 4'-cyano-α',α',α'-trifluoro-3-(4-fluorophenylsulphonyl)-2-hydroxy-2-methylpropiono-*m*-toluidide.

23. The use according to claim 22, wherein >50% of the 4'-cyano-α',α',α'-trifluoro-3-(4-fluorophenylsulphonyl)-2-hydroxy-2-methylpropiono-*m*-toluidide is provided in the form of the R-enantiomer.

24. The use according to any one of claims 16 to 23, wherein the ratio of 4'-cyano-α',α',α'-trifluoro-3-(4-fluorophenylsulphonyl)-2-hydroxy-2-methylpropiono-*m*-toluidide: PVP by weight is from 1:0.25 to 1:10.

25. The use according to claim 24, wherein the ratio of-4'-cyano-α',α',α'-trifluoro-3-(4-fluorophenylsulphonyl)-2-hydroxy-2-methylpropiono-*m*-toluidide: PVP by weight is 1:>3 to 1:10.

26. The use according to any one of claims 16 to 25, wherein the solid dispersion includes a wetting agent.

27. The use according to claim 24, wherein the ratio of 4'-cyano-α',α',α'-trifluoro-3-(4-fluorophenylsulphonyl)-2-hydroxy-2-methylpropiono-*m*-toluidide: PVP by weight is 1:0.25 to 1:≤3 and the solid dispersion includes a wetting agent.

28. The use according to any one of claims 16 to 27, wherein about ≥50%, ≥60%, ≥65%, ≥70%, ≥80%, ≥85%, ≥90%, ≥95%, ≥98% or ≥99% of the 4'-cyano-α',α',α'-trifluoro-3-(4-fluorophenylsulphonyl)-2-hydroxy-2-methylpropiono-*m*-toluidide is provided in the form of the R-enantiomer.

29. The use according to claim 28, wherein substantially 100% of the 4'-cyano-α',α',α'-trifluoro-3-(4-fluorophenylsulphonyl)-2-hydroxy-2-methylpropiono-*m*-toluidide is provided in the form of the R-enantiomer.

30. The use according to any one of claims 16 to 29, wherein the PVP has a K-value ≤90.

31. The use according to claim 30, wherein the 4'-cyano-α',α',α'-trifluoro-3-(4-fluorophenylsulphonyl)-2-hydroxy-2-methylpropiono-*m*-toluidide is in solid dispersion with PVP K-25.

32. A method for enhancing the storage stability of the 4'-cyano-α',α',α'-trifluoro-3 (4-fluorophenylsulphonyl)-2-hydroxy-2-methylpropiono-*m*-toluidide in a formulation comprising 4'-cyano-α',α',α'-trifluoro-3-(4-fluorophenylsulphonyl)-2-hydroxy-2-methylpropiono-*m*-toluidide in a solid dispersion with PVP, comprising use of 4'-cyano-α',α',α'-trifluoro-3-(4-fluorophenylsulphonyl)-2-hydroxy-2-methylpropiono-*m*-toluidide wherein >50% of it is provided in the form of the R-enantiomer.

33. The formulation, dose, solid dispersion, use or method according to any of the preceding claims wherein, at least 20% of the 4'-cyano-α',α',α'-trifluoro-3-(4-fluorophenylsulphonyl)-2-hydroxy-2-methylpropiono-*m*-toluidide is present in amorphous form.

## Patentansprüche

1. Pharmazeutische Formulierung zur mucosalen Verabreichung an einen Patienten, die 4'-Cyano-α',α',α'-trifluor-3-(4-fluorphenylsulfonyl)-2-hydroxy-2-methylpropiono-*m*-toluidid in fester Dispersion mit PVP enthält.

2. Pharmazeutische Formulierung nach Anspruch 1, bei der >50% des 4'-Cyano-α',α',α'-trifluor-3-(4-fluorphenylsulfonyl)-2-hydroxy-2-methylpropiono-*m-*toluidids in Form des R-Enantiomers bereitgestellt werden.

3. An einen Patienten zur Behandlung und/oder Verringerung des Risikos von Prostatakrebs bei dem Patienten mucosal verabreichbare pharmazeutische Tagesdosis von 4'-Cyano-α',α',α'-trifluor-3-(4-fluorphenylsulfonyl)-2-hydroxy-2-methylpropiono-*m-*toluidid, die 10 bis 1500 mg 4'-Cyano-α',α',α'-trifluor-3-(4-fluorphenylsulfonyl)-2-hydroxy-2-methylpropiono-*m*-toluidid in fester Dispersion mit PVP enthält.

4. Pharmazeutische Tagesdosis von 4'-Cyano-α',α',α'-trifluor-3-(4-fluorphenylsulfonyl)-2-hydroxy-2-methylpropiono-*m*-toluidid nach Anspruch 3, bei der >50% des 4'-Cyano-α',α',α'-trifluor-3-(4-fluorphenylsulfonyl)-2-hydroxy-2-methylpropiono-*m-*toluidids in Form des R-Enantiomers bereitgestellt werden.

5. Formulierung nach Anspruch 1 oder 2 oder Dosis nach Anspruch 3 oder 4, bei der das Gewichtsverhältnis von 4'-Cyano-α',α',α'-trifluor-3-(4-fluorphenylsulfonyl)-2-hydroxy-2-methylpropiono-*m*-toluidid zu PVP 1:0,25 bis 1:10 beträgt.

6. Formulierung oder Dosis nach Anspruch 5, bei der das Gewichtsverhältnis von 4'-Cyano-α',α',α'-trifluor-3-(4-fluorphenylsulfonyl)-2-hydroxy-2-methylpropiono-*m*-toluidid zu PVP 1:>3 bis 1:10 beträgt.

7. Formulierung oder Dosis nach einem der vorhergehenden Ansprüche, bei der die feste Dispersion ein Netzmittel enthält.

8. Formulierung oder Dosis nach Anspruch 5, bei der das Gewichtsverhältnis von 4'-Cyano-α',α',α'-trifluor-3-(4-fluorphenylsulfonyl)-2-hydroxy-2-methylpropiono-*m*-toluidid zu PVP 1:0,25 bis 1:≤3 beträgt und die feste Dispersion ein Netzmittel enthält.

9. Formulierung oder Dosis nach einem der vorhergehenden Ansprüche, bei der etwa ≥50%, ≥60%, ≥65%, ≥70%, ≥80%, ≥85%, ≥90%, ≥95%, ≥98% oder ≥99% des 4'-Cyano-α',α',α'-trifluor-3-(4-fluorphenylsulfonyl)-2-hydroxy-2-methylpropiono-*m*-toluidids in Form des R-Enantiomers bereitgestellt werden.

10. Formulierung oder Dosis nach Anspruch 9, bei der weitgehend 100% des 4'-Cyano-α',α',α'-trifluor-3-(4-fluorphenylsulfonyl)-2-hydroxy-2-methylpropiono-*m*-toluidids in Form des R-Enantiomers bereitgestellt werden.

11. Formulierung oder Dosis nach einem der vorhergehenden Ansprüche, bei der das PVP einen K-Wert ≤90 aufweist.

12. Formulierung oder Dosis nach Anspruch 11, bei der das 4'-Cyano-α',α',α'-trifluor-3-(4-fluorphenylsulfonyl)-2-hydroxy-2-methylpropiono-*m*-toluidid in fester Dispersion mit PVP K-25 vorliegt..

13. Feste Dispersion von PVP mit 4'-Cyano-α',α',α'-trifluor-3-(4-fluorphenylsulfonyl)-2-hydroxy-2-methylpropiono-*m*-toluidid zur Verwendung als Arzneimittel.

14. Feste Dispersion nach Anspruch 13, bei der >50% des 4'-Cyano-α',α',α'-trifluor-3-(4-fluorphenylsulfonyl)-2-hydroxy-2-methylpropiono-*m*-toluidids in Form des R-Enantiomers bereitgestellt werden.

15. Feste Dispersion nach Anspruch 13 oder 14, bei der die feste Dispersion ein Netzmittel enthält.

16. Verwendung von PVP in fester Dispersion mit 4'-Cyano-α',α',α'-trifluor-3-(4-fluorphenylsulfonyl)-2-hydroxy-2-methylpropiono-*m*-toluidid bei der Herstellung eines an einen Patienten mucosal verabreichbaren Arzneimittels zur Erhöhung der Bioverfügbarkeit von 4'-Cyano-α',α',α'-trifluor-3-(4-fluorphenylsulfonyl)-2-hydroxy-2-methylpropiono-*m*-toluidid bei dem Patienten.

17. Verwendung von PVP in fester Dispersion mit 4'-Cyano-α',α',α'-trifluor-3-(4-fluorphenylsulfonyl)-2-hydroxy-2-methylpropiono-*m*-toluidid bei der Herstellung eines an einen Patienten mucosal verabreichbaren Arzneimittels zur Verringerung der Variabilität der Plasmakonzentrationen von 4'-Cyano-α',α',α'-trifluor-3-(4-fluorphenylsulfonyl)-2-hydroxy-2-methylpropiono-*m*-toluidid zwischen verschiedenen Patienten.

18. Verwendung von PVP in fester Dispersion mit 4'-Cyano-α',α',α'-trifluor-3-(4-fluorphenylsulfonyl)-2-hydroxy-2-methylpropiono-*m*-toluidid bei der Herstellung einer an einen Patienten mucosal verabreichbaren pharmazeutischen Formulierung zur Erhöhung der Bioverfügbarkeit von 4'-Cyano-α',α',α'-trifluor-3-(4-fluorphenylsulfonyl)-2-hydroxy-2-methylpropiono-*m*-toluidid bei dem Patienten und/oder Verringerung der Variabilität der Plasmakonzentrationen von 4'-Cyano-α',α',α'-trifluor-3-(4-fluorphenylsulfonyl)-2-hydroxy-2-methylpropiono-*m*-toluidid zwischen verschiedenen Patienten.

19. Verwendung von PVP in fester Dispersion mit 4'-Cyano-α',α',α'-trifluor-3-(4-fluorphenylsulfonyl)-2-hydroxy-2-methylpropiono-*m*-toluidid bei der Herstellung eines an einen Patienten mucosal verabreichbaren Arzneimittels zur Behandlung und/oder Verringerung des Risikos von Prostatakrebs bei dem Patienten.

20. Verwendung nach einem der Ansprüche 16-19, bei dem >50% des 4'-Cyano-α',α',α'-trifluor-3-(4-fluorphenylsulfonyl)-2-hydroxy-2-methylpropiono-*m-*toluidids in Form des R-Enantiomers bereitgestellt werden.

21. Verwendung von PVP in fester Dispersion mit 4'-Cyano-a',a',a'-trifluor-3-(4-fluorphenylsulfonyl)-2-hydroxy-2-methylpropiono-*m*-toluidid, bei der >50% des 4'-Cyano-α',α',α'-trifluor-3-(4-fluorphenylsulfonyl)-2-hydroxy-2-methylpropiono-*m-*toluidids in Form des R-Enantiomers bereitgestellt werden, bei der Herstellung einer pharmazeutischen Formulierung zur Erhöhung der Lagerstabilität des 4'-Cyano-α',α',α'-trifluor-3-(4-fluorphenylsulfonyl)-2-hydroxy-2-methylpropiono-*m*-toluidids in der Formulierung.

22. Verwendung nach Anspruch 19, bei der das Arzneimittel als Tagesdosis von 4'-Cyano-α',α',α'-trifluor-3-(4-fluorphenylsulfonyl)-2-hydroxy-2-methylpropiono-*m*-toluidid bereitgestellt wird und 10 bis 1500 g 4'-Cyano-α',α',α'-trifluor-3-(4-fluorphenylsulfonyl)-2-hydroxy-2-methylpropiono-*m-*toluidid enthält.

23. Verwendung nach Anspruch 22, bei der >50% des 4'-Cyano-α',α',α'-trifluor-3-(4-fluorphenylsulfonyl)-2-hydroxy-2-methylpropiono-*m*-toluidids in Form des R-Enantiomers bereitgestellt werden.

24. Verwendung nach einem der Ansprüche 16 bis 23, bei der das Gewichtsverhältnis von 4'-Cyano-α',α',α'-trifluor-3-(4-fluorphenylsulfonyl)-2-hydroxy-2-methylpropiono-*m*-toluidid zu PVP 1:0,25 bis 1:10 beträgt.

25. Verwendung nach Anspruch 24, bei der das Gewichtsverhältnis von 4'-Cyano-α',α',α'-trifluor-3-(4-fluorphenylsulfonyl)-2-hydroxy-2-methylpropiono-*m*-toluidid zu PVP 1:>3 bis 1:10 beträgt.

26. Verwendung nach einem der Ansprüche 16 bis 25, bei der die feste Dispersion ein Netzmittel enthält.

27. Verwendung nach Anspruch 24, bei der das Gewichtsverhältnis von 4'-Cyano-α',α',α'-trifluor-3-(4-fluorphenylsulfonyl)-2-hydroxy-2-methylpropiono-*m*-toluidid zu PVP 1:0,25 bis 1:≤3 beträgt und die feste Dispersion ein Netzmittel enthält.

28. Verwendung nach einem der Ansprüche 16 bis 27, bei der etwa ≥50%, ≥60%, ≥65%, ≥70%, ≥80%, ≥85%, ≥90%, ≥95%, ≥98% oder ≥99% des 4'-Cyano-α',α',α'-trifluor-3-(4-fluorphenylsulfonyl)-2-hydroxy-2-methylpropiono-*m*-toluidids in Form des R-Enantiomers bereitgestellt werden.

29. Verwendung bei der weitgehend 100% des 4'-Cyano-α',α',α'-trifluor-3-(4-fluorphenylsulfonyl)-2-hydroxy-2-methylpropiono-*m*-toluidids in Form des R-Enantiomers bereitgestellt werden.

30. Verwendung nach einem der Ansprüche 16 bis 29, bei der das PVP einen K-Wert ≤90 aufweist.

31. Verwendung nach Anspruch 30, bei der das 4'-Cyano-α',α',α'-trifluor-3-(4-fluorphenylsulfonyl)-2-hydroxy-2-methylpropiono-*m*-toluidid in fester Dispersion mit PVP K-25 vorliegt.

32. Verfahren zur Verbesserung der Lagerstabilität des 4'-Cyano-α',α',α'-trifluor-3-(4-fluorphenylsulfonyl)-2-hydroxy-2-methylpropiono-*m*-toluidids in einer Formulierung, die 4'-Cyano-α',α',α'-trifluor-3-(4-fluorphenylsulfonyl)-2-hydroxy-2-methylpropiono-*m*-toluidid in fester Dispersion mit PVP enthält, unter Verwendung von 4'-Cyano-α',α',α'-trifluor-3-(9-fluorphenylsulfonyl)-2-hydroxy-2-methylpropiono-*m*-toluidid, bei dem >50% davon in Form des R-Enantiomers bereitgestellt werden.

33. Formulierung, Dosis, feste Dispersion, Verwendung oder Verfahren nach einem der vorhergehenden Ansprüche, worin mindestens 20% des 4'-Cyano-α',α',α'-trifluor-3-(4-fluorphenylsulfonyl)-2-hydroxy-2-methylpropiono-*m*-toluidids in amorpher Form vorliegen.

## Revendications

1. Formulation pharmaceutique destinée à une administration par voie muqueuse à un patient, la formulation comprenant le 4'-cyano-α',α',α'-trifluoro-3-(4-fluorophénylsulfonyl)-2-hydroxy-2-méthylpropiono-*m*-toluidiure en dispersion solide avec la PVP.

2. Formulation pharmaceutique selon la revendication 1, **caractérisée en ce que** > à 50% du 4'-cyano-α',α',α'-trifluoro-3-(4-fluorophénylsulfonyl)-2-hydroxy-2-méthylpropiono-*m*-toluidiure se présentent sous forme de l'énantiomère R.

3. Dose pharmaceutique journalière de 4'-cyano-α',α',α'-trifluoro-3-(4-fluorophénylsulfonyl)-2-hydroxy-2-méthylpropiono-*m*-toluidiure administrable par voie muqueuse à un patient pour traiter et/ou réduire le risque d'un cancer de la prostate chez le patient, **caractérisée en ce que** la dose comprend de 10 à 1500 mg de 4'-cyano-α',α',α'-trifluoro-3-(4-fluorophénylsulfonyl)-2-hydroxy-2-méthylpropiono-*m*-toluidiure dans une dispersion solide avec la PVP.

4. Dose pharmaceutique journalière de 4'-cyano-(α',α',α'-trifluoro-3-(4-fluorophénylsulfonyl)-2-hydroxy-2-méthylpropiono-*m*-toluidiure selon la revendication 3, **caractérisée en ce que** > à 50% du 4'-cyano-α',α',α'-trifluoro-3-(4-fluorophénylsulfonyl)-2-hydroxy-2-méthylpropiono-*m*-toluidiure se présentent sous forme de l'énantiomère R.

5. Formulation selon la revendication 1 ou 2 ou dose selon la revendication 3 ou 4, **caractérisée en ce que** le rapport 4'-cyano-α',α',α'-trifluoro-3-(4-fluorophénylsulfonyl)-2-hydroxy-2-méthylpropiono-*m*-toluidiure:PVP en poids est de 1:0,25 à 1:10.

6. Formulation ou dose selon la revendication 5, **caractérisée en ce que** le rapport 4'-cyano-α',α',α'-trifluoro-3-(4-fluorophénylsulfonyl)-2-hydroxy-2-méthylpropiono-*m*-toluidiure:PVP en poids est de 1:> à 3 à 1:10.

7. Formulation ou dose selon l'une quelconque des revendications précédentes, **caractérisée en ce que** la dispersion solide comprend un agent mouillant.

8. Formulation ou dose selon la revendication 5, **caractérisée en ce que** le rapport 4'-cyano-α',α',α'-trifluoro-3-(4-fluorophénylsulfonyl)-2-hydroxy-2-méthylpropiono-*m*-toluidiure:PVP en poids est de 1:0,25 à 1:≤ à 3 et la dispersion solide comprend un agent mouillant.

9. Formulation ou dose selon l'une quelconque des revendications précédentes, **caractérisée en ce que** environ ≥ à 50%, ≥ à 60%, ≥ à 65%, ≥ à 70%, ≥ à 80%, ≥ à 85%, ≥ à 90%, ≥ à 95%, ≥ à 98% ou ≥ à 99% du 4'-cyano-α',α',α'-trifluoro-3-(4-fluorophénylsulfonyl)-2-hydroxy-2-méthylpropiono-*m*-toluidiure se présentent sous forme de l'énantiomère R.

10. Formulation ou dose selon la revendication 9, **caractérisée en ce que** substantiellement 100% du 4'-cyano-α',α',α'-trifluoro-3-(4-fluorophénylsulfonyl)-2-hydroxy-2-méthylpropiono-*m*-toluidiure se présentent sous forme de l'énantiomére R.

11. Formulation ou dose selon l'une quelconque des revendications précédentes **caractérisée en ce que** la PVP présente une valeur de K ≤ à 90.

12. Formulation ou dose selon la revendication 11, **caractérisée en ce que** le 4'-cyano-α',α',α'-trifluoro-3-(4-fluorophénylsulfonyl)-2-hydroxy-2-méthylpropiono-*m*-toluidiure est en dispersion solide avec la PVP K-25.

13. Dispersion solide de PVP avec le 4'-cyano-α',α',α'-trifluoro-3-(4-fluorophénylsulfonyl)-2-hydroxy-2-méthylpropiono-*m*-toluidiure, destinée à être utilisée en tant que médicament.

14. Dispersion solide selon la revendication 13, **caractérisée en ce que** > à 50% du 4'-cyano-α',α',α'-trifluoro-3-(4-fluorophénylsulfonyl)-2-hydroxy-2-méthylpropiono-*m*-toluidiure se présentent sous forme de l'énantiomère R.

15. Dispersion solide selon la revendication 13 ou la revendication 14, **caractérisée en ce que** la dispersion solide comprend un agent mouillant.

16. Utilisation de la PVP en dispersion solide avec le 4'-cyano-α',α',α'-trifluoro-3-(4-fluorophénylsulfonyl)-2-hydroxy-2-méthylpropiono-*m*-toluidiure dans la fabrication d'un médicament administrable par voie muqueuse à un patient, pour accroître la biodisponibilité du 4'-cyano-α',α',α'-trifluoro-3-(4-fluorophénylsulfonyl)-2-hydroxy-2-méthylpropiono-*m*-toluidiure chez le patient.

17. Utilisation de la PVP en dispersion solide avec le 4'-cyano-α',α',α'-trifluoro-3-(4-fluorophénylsulfonyl)-2-hydroxy-2-méthylpropiono-*m*-toluidiure dans la fabrication d'un médicament administrable par voie muqueuse à des patients, pour réduire la variabilité inter-patient dans les concentrations plasmatiques du 4'-cyano-α',α',α'-trifluoro-3-(4-fluorophénylsulfonyl)-2-hydroxy-2-méthylpropiono-*m*-toluidiure.

18. Utilisation de la PVP en dispersion solide avec le 4'-cyano-α',α',α'-trifluoro-3-(4-fluorophénylsulfonyl)-2-hydroxy-2-méthylpropiono-*m*-toluidiure dans la fabrication d'une formulation pharmaceutique administrable par voie muqueuse à un patient, pour accroître la biodisponibilité du 4'-cyano-α',α',α'-trifluoro-3-(4-fluorophénylsulfonyl)-2-hydroxy-2-méthylpropiono-*m*-toluidiure chez le patient et/ou réduire la variabilité inter-patient dans les concentrations plasmatiques du 4'-cyano-α',α',α'-trifluoro-3-(4-fluorophénylsulfonyl)-2-hydroxy-2-méthylpropiono-*m*-toluidiure.

19. Utilisation de la PVP en dispersion solide avec le 4'-cyano-α',α',α'-trifluoro-3 -(4-fluorophénylsulfonyl)-2-hydroxy-2-méthylpropiono-*m*-toluidiure dans la fabrication d'un médicament administrable par voie muqueuse à un patient, pour traiter et/ou réduire le risque du cancer de la prostate chez le patient.

20. Utilisation selon l'une quelconque des revendications 16-19, **caractérisée en ce que** > à 50% du 4'-cyano-α',α',α'-trifluoro-3-(4-fluorophénylsulfonyl)-2-hydroxy-2-méthylpropiono-*m*-toluidiure se présentent sous forme de l'énantiomère R.

21. Utilisation de la PVP en dispersion solide avec le 4'-cyano-α',α',α'-trifluoro-3-(4-fluorophénylsulfonyl)-2-hydroxy-2-méthylpropiono-*m*-toluidiure, **caractérisée en ce que** > à 50% du 4'-cyano-α',α',α'-trifluoro-3-(4-fluorophénylsulfonyl)-2-hydroxy-2-méthylpropiono-*m*-toluidiure se présentent sous forme de l'énantiomère R dans la fabrication d'une formulation pharmaceutique, pour améliorer la stabilité au cours du stockage du 4'-cyano-α',α',α'-trifluoro-3-(4-fluorophénylsulfonyl)-2-hydroxy-2-méthylpropiono-*m*-toluidiure dans la formulation.

22. Utilisation selon la revendication 19, **caractérisée en ce que** le médicament se présente en dose journalière du 4'-cyano-α',α',α'-trifluoro-3-(4-fluorophénylsulfonyl)-2-hydroxy-2-méthylpropiono-*m*-toluidiure et comprend de 10 à 1500 mg de 4'-cyano-α',α',α'-trifluoro-3-(4-fluorophénylsulfonyl)-2-hydroxy-2-méthylpropiono-*m*-toluidiure.

23. Utilisation selon la revendication 22, **caractérisée en ce que** > 50% du 4'-cyano-α',α',α'-trifluoro-3-(4-fluorophénylsulfonyl)-2-hydroxy-2-méthylpropiono-*m*-toluidiure se présentent sous forme de l'énantiomère R.

24. Utilisation selon l'une quelconque des revendications 16 à 23, **caractérisée en ce que** le rapport 4'-cyano-α',α',α'-trifluoro-3-(4-fluorophénylsulfonyl)-2-hydroxy-2-méthylpropiono-*m*-toluidiure:PVP en poids est de 1:0,25 à 1:10.

25. Utilisation selon la revendication 24, **caractérisée en ce que** le rapport 4'-cyano-α',α',α'-trifluoro-3-(4-fluorophénylsulfonyl)-2-hydroxy-2-méthylpropiono-*m*-toluidiure:PVP en poids est de 1:> à 3 à 1:10.

26. Utilisation selon l'une quelconque des revendications 16 à 25, **caractérisée en ce que** la dispersion solide comprend un agent mouillant.

27. Utilisation selon la revendication 24, **caractérisée en ce que** le rapport 4'-cyano-α',α',α'-trifluoro-3-(4-fluorophénylsulfonyl)-2-hydroxy-2-méthylpropiono-*m*-toluidiure:PVP en poids est de 1:0,25 à 1:≤ à 3 et la dispersion solide comprend un agent mouillant.

28. Utilisation selon l'une quelconque des revendications 16 à 27, **caractérisée en ce que** environ ≥ à 50%, ≥ à 60%, ≥ à 65%, ≥ à 70%, ≥ à 80%, ≥ à 85%, ≥ à 90%, ≥ à 95%, ≥ à 98% ou ≥ à 99% du 4'-cyano-α',α',α'-trifluoro-3-(4-fluorophénylsulfonyl)-2-hydroxy-2-méthylpropiono-*m*-toluidiure se présentent sous forme de l'énantiomère R.

29. Utilisation selon la revendication 28, **caractérisée en ce que** substantiellement 100% du 4'-cyano-α',α',α'-trifluoro-3-(4-fluorophénylsulfonyl)-2-hydroxy-2-méthylpropiono-*m*-toluidiure se présentent sous forme de l'énantiomère R.

30. Utilisation selon l'une quelconque des revendications 16 à 29, **caractérisée en ce que** la PVP présente une valeur de K ≤ à 90.

31. Utilisation selon la revendication 30, **caractérisée en ce que** le 4'-cyano-α',α',α'-trifluoro-3-(4-fluorophénylsulfonyl)-2-hydroxy-2-méthylpropiono-*m*-toluidiure est en dispersion solide avec la PVP K-25.

32. Procédé d'amélioration de la stabilité au stockage du 4'-cyano-α',α',α'-trifluoro-3-(4-fluorophénylsulfonyl)-2-hydroxy-2-méthylpropiono-*m*-toluidiure dans une formulation comprenant le 4'-cyano-α',α',α'-trifluoro-3-(4-fluorophénylsulfonyl)-2-hydroxy-2-méthylpropiono-*m*-toluidiure en dispersion solide avec la PVP comprenant l'utilisation de 4'-cyano-α',α',α'-trifluoro-3-(4-fluorophénylsulfonyl)-2-hydroxy-2-méthylpropiono-*m*-toluidiure, **caractérisé en ce que** > à 50% de celui-ci se présentent sous forme de l'énantiomère R.

33. Formulation, dose, dispersion solide, utilisation ou procédé selon l'une quelconque des revendications précédentes, **caractérisés en ce que** au moins 20% du 4'-cyano-α',α',α'-trifluoro-3-(4-fluorophénylsulfonyl)-2-hydroxy-2-méthylpropiono-*m*-toluidiure se présentent sous forme amorphe.
